# EUROPEAN PATENT APPLICATION

(11) **EP 4 497 327 A1**
(43) Date of publication of application: **29.01.2025**
(21) Application number: 24386088.9
(22) Date of filing: 18.07.2024
(51) Int. Cl.: A01N 31/02, A01N 37/44, A01N 59/02, A01P 3/00, A01P 21/00, A23J 3/34, C05F 11/00, C07C 227/28, C07K 14/415

(54) **METHOD FOR THE PRODUCTION OF PLANT BIOSTIMULANT AND THE PRODUCT THEREOF**

(30) Priority: 27.07.2023 GR 20230100620
(71) Applicant: EVYP EE, 57022 Thessaloniki (GR)
(72) Inventor: Aikaterini, Michailidou, 55236 Panorama Thessalonikis (GR)

(57) **Abstract**

The invention relates to a method of producing a plant biostimulant and the product thereof. The resulting product achieves improved extraction/hydrolysis efficiency, using sulfuric acid. It gives a high number of sulphated L amino acids of plant origin in a high total content of 16,78 % w/w, a number of low molecular weight peptides and contains natural Betaine, Mannitol (osmolytes) and sterols. The product obtained has a zero-chlorine content. Therefore, a higher product dispersibility through plant tissues is achieved. The final product can be used as a supplement for the enhancement and growth of all conventional and organic crops.

## Description

### STATE OF THE ART

The invention relates to the field of human needs and more particularly to the field of agriculture. In particular, it relates to the field of biocides, insect repellents and plant growth regulators. In particular, it relates to a plant biostimulant, as well as to a method of producing said biostimulant. It relates to an innovative biostimulant - a plant growth enhancement supplement and its method of production.

In particular, the invention relates to a method for obtaining from genetically unmodified plant raw materials, organic free L-amino acids enriched with sulfur, suitable also for organic cultivation.

It also contains peptides that have been shown to have a signaling function that regulates plant physiology by acting as signaling molecules and influencing the action of phytohormones against abiotic stress.

The final product is a chlorine-free solution of sulphated free L-amino acids and peptides.

### BACKGROUND OF THE INVENTION

The method of producing plant biostimulant disclosed in the present invention, as well as the resulting product, have not been disclosed in the prior art.

Intensive growing conditions, such as large number of plants per unit area, high humidity conditions with insufficient ventilation in greenhouses, excessive use of fertilizers, stress due to climate change, make plants vulnerable to fungal diseases of the root system and leaf surface, weaken their immune system and expose them to a variety of toxicities. In addition, intensive cultivation conditions, mainly due to excessive fertilization, render the soil unable to absorb nutrients. These problems are usually addressed by the use of biostimulants and fertilizer products containing chlorine in various proportions.

Chlorine plays an important role in many physiological processes in plants and is a micronutrient in terms of plant nutrition. The average chlorine content in plants is 2-20 mg/g dry weight. The chlorine requirements of most plants are 10 to 100 times lower. Therefore, at the application level, chlorine surplus is more often found than deficit. Chlorine deficiency causes salinity, increased conductivity of nutrient solutions and chlorination of soils.

To date, solutions to reduce chlorination are quite limited. The use of products with as little chlorine as possible in their composition is the only one that can be applied. Products with zero chlorine content are few in number and more expensive than conventional products.

The increasing use of biostimulants can help significantly, provided they contain little or no chlorine. The desire to completely eliminate the use of chlorine has led to the creation of the invention disclosed herein.

At the same time, many amino acid and sulfur-containing biostimulants are the result of secondary mixing of amino acid solutions with sulfur solutions, which result in potential phase separation of the application solution and in many cases limited use efficiency. Further, there are several state-of-the-art products containing amino acids, the main component of which is, of plant origin, a protein similar to the protein used in the present invention, but many also contain proteins of animal origin or only animal origin. Finally, even among the various methods of producing products containing amino acids of plant origin, such as from maize protein, there are significant disadvantages and differences with the method proposed herein.

Already known inventions include EP2537823, which discloses a plant growth enhancer containing chlorine in small, but non-harmful amounts. Similarly, in PL 232367B1, although natural raw materials are used, keratin, which is a protein of animal origin, is also used. Further known is document CN102924181A, which although using maize protein, applies different steps, such as leaching of the raw material for pre-treatment and fermentation and then neutralization. Similarly, the product presented in CN 110089615A also uses maize, wheat and soybean protein components, but adopts enzymatic hydrolysis. CN 1114146A uses phosphoric acid and sulphuric acid simultaneously. In document CN1334260A a conventional source of inorganic sulphur is used to achieve the final result. Finally, document EP2537823 uses hydrochloric acid, for which a comparative table of results of its use is provided, in relation to the present invention.

It is thus an object of the present invention to advantageously address the aforementioned disadvantages and shortcomings of the prior art, by proposing a plant biostimulant which is completely free of chlorine.

It is a further object of the present invention to provide a method of producing a plant biostimulant, which uses sulfuric acid for the acid hydrolysis of plant, non-GMO proteins and results in a product of sulfurized amino acids and post-translationally modified peptides, of low molecular weight and sterols. The resulting biostimulant is a chlorine-free solution of sulfated free L-amino acids and peptides.

It is a further object of the present invention to provide a plant biostimulant, which contains only proteins of plant origin.

It is a further object of the invention to provide a method for obtaining organic free L-amino acids, from genetically unmodified sulfur-enriched plant raw materials, suitable for biological cultivation.

It is a further object of the invention to disclose a plant biostimulant wherein the final product is a non-toxic solution, containing natural Betaine, Mannitol (osmolytes) and sterols.

Another object of the invention is the production of a biostimulant in an economical and time-shortened manner, which contains only free L amino acids enriched with sulfur and peptides, homogeneous, super-concentrated, with complete solubility and which contains chemically incorporated sulfur.

An advantage of the present invention is also that it contains an increased proportion of Proline, which leads to enhanced tolerance to cold stress and a significant increase in crop growth and yield under environmental adversity.

An additional advantage of the present invention is that it contains a highly increased percentage of glutamic acid, which is a precursor to the synthesis of proteins, polypeptides, amino acids, non-protein amino acids (gamma-aminobutyric acid, GABA), antioxidant tripeptides (glutathione, GSH) and chlorophyll. It further increases the concentration of flavonoids, especially phenols, glucosinoids and chlorophyll, especially in plants of the crabapple family.

Another advantage of the invention is that it enhances the process of photosynthesis, stimulates basic metabolic functions, increases production and improves plant productivity.

Yet another advantage is that it enhances the natural fungistatic activity of plants thanks to its bioabsorbable sulfur-containing and biosynthesis of complex secondary metabolites, such as sulfur-containing defense chemicals (SDCs) and antimicrobial peptides (AMPs). Thus, the plant activates its natural defense organisms, making it resistant to plant diseases.

Significant further advantages of the invention's biostimulant are that it increases the concentration and quality of protein, especially in cereal seeds, by altering the percentage of phytic acid and plant ionomycin. It also provides the plant with the basic building blocks it needs, so it does not have to consume energy to synthesize its own protein.

An additional advantage is that it helps the process of pollination and fruit formation, as the specific and important phases of growth require high levels of amino acids.

Another advantage of the method is that no residues or waste is produced during its application, as all the ingredients used in the method are used in their pure form, without pre-treatment, in the exact proportions required.

The sulphate portion of the sulphurated amino acids produced, provides stability in the production of endogenous peptide hormones vital for plant growth.

The pH of the aqueous solution at the recommended rates is the most suitable for the absorption of the nutrients during the trans-water spray.

The final product is a model of a circular economy, since it utilizes plant-derived byproducts to produce higher value-added amino acid and protein biopolymers, which end product is returned to the crops.

In addition, it improves nitrogen efficiency. It is essential for the synthesis of sulphur-containing amino acids such as cystine, cysteine and methionine and for the effect on overall protein synthesis. It further activates important enzymes in energy and fatty acid metabolism and is a component of chloroplast protein. It is important for the formation of secondary sulphur-containing plant substances such as vitamins, antioxidants, carotenoids; it is also a component of vitamin B1 (cereal grains, legumes); it is important for the production of plant defense substances (phytoalexins, glutathione). Lastly, it prevents chlorosis, has a zero-chlorine content and shields the plant, giving it therapeutic properties against pathogens (fungicidal action).

These and other objects, features and advantages of the invention will become apparent from the detailed description below.

### DETAILED DESCRIPTION OF A PREFERRED APPLICATION

The invention will be made apparent to those skilled in the art by reference to examples of application, to which it is not limited.

As is well known, amino acids are an essential building block of all organisms and are widely used as a plant growth supplement and as additives in animal feed, nutritional products and cosmetics. Elemental sulphur-enriched amino acids are known to help plants to absorb appropriate and essential nutrients from the soil, as they are involved in the transmission of signals/instructions to the plant cell.

The present invention is an amino acid solution enriched with sulfur, with the source of the sulfur coming exclusively from the hydrolysis medium (sulfuric acid), which is used as the initial key ingredient of the composition in a specific proportion, rather than an inorganic source of sulfur added in the process. The present invention does not contain chlorine, achieves improved extraction efficiency and thus gives a higher number of amino acids, higher total content by weight and a plurality of low molecular weight peptides. Consequently, a higher product dispersibility through plant tissues is achieved. The present invention naturally solves the problem of over-fertilization and continuous use of chlorine-containing products, as it is a method of producing free L-amino acids and peptides of plant origin, the main components of which are corn, wheat and soy protein. In the invention disclosed herein, 18% sulfuric acid is used versus 20% hydrochloric acid applied in EP 2537823 (less acid).

In the present invention the sulfuric acid performs two distinct roles. Firstly, it is used as a means of hydrolysis and breakdown of plant proteins, and further provides a very essential macronutrient for plant growth, sulphur. Sulphur is a constituent of certain amino acids such as cystine, thiamine and methionine and is involved in protein synthesis, is a component of coenzyme A, vitamin B1 and ferredoxin, as well as glycosides. In addition, sulphur exhibits fungicidal activity and is used commercially in various fungicidal admixtures.

In particular, the method of producing amino acids and peptides of plant origin preferably uses, but not limited to, 18 % w/w corn gluten, 5,5 % w/w wheat gluten, 6,5 % w/w soy protein, 18 % w/w sulphuric acid and water 52% w/w. The above raw materials are placed in a reactor at the same time, without any prior preparation or treatment. The materials remain in the reactor under conditions of continuous, constant agitation, preferably, but not limited to, a temperature of 110° Celsius, at a pressure of 4 bar for a period of 16 hours. The process described above results directly in the production of a final product containing free amino acids and peptides enriched with elemental sulphur.

The conditions of the process as described above are ideal for the production of the desired product. The above method produces the same product even at a temperature deviation of ± 2 %, i.e. from 107° to 113° Celsius, a pressure deviation of ± 5 %, i.e. from 3,8 to 4,2 bar, and for a minimum period of 14 hours and a maximum period of 18 hours. Similarly, the method can be applied in the same way and produce a product with similar properties when raw materials are used, in the following ranges. Accordingly, corn gluten can be used in a proportion of from 15 % to 21 %, wheat gluten in a proportion of from 3,5 % to 7,5 %, soy protein in a proportion of from 4,5 % to 8,5 %, sulphuric acid in a proportion of from 16 % to 20 % and water in a proportion of from 50 % to 54 %.

The physical state of the product is liquid with a density of 1,2 g/ml, brown to dark brown in colour with the characteristic odour of the protein hydrolysis process and sulphur.

The application of the process described above always produces a stable end product, which has the following composition in terms of the percentage (%) of carbon to nitrogen (C/N) ratio:

| | % b.w. of product | N% w/w (minimum) | C% w/w (minimum) |
|---|---|---|---|
| Amino acids | 16,78 | 2,5 | 7,3 |
| Peptides | 1,97 | 0,5 | 0,9 |
| Sugars, hydrolysis residues | 28,55 | - | 18,6 |
| Water | 52,7 | | |
| C/N ratio = 8,93 | | | |

The table below lists the laboratory results obtained after chemical analysis of the product, compared to document EP 2537823, which is considered to be more relevant to the present invention.

| | | EP 2 537 823 B1 (Amino16) | Current invention |
|---|---|---|---|
| | | % w/w of product | % w/w of product |
| 1 | Alanine Ala | | |
| 2 | Arginine Arg | 0,75 | 0,51 |
| 3 | Aspartic acid | 1,10 | 1,77 |
| 4 | Glutamic acid Glu | 1,33 | 3,75 |
| 5 | Glycine Gly | 0,32 | 0,52 |
| 6 | Histidine His | 0,31 | 0,32 |
| 7 | Isoleucine Ile | 0,75 | 0,42 |
| 8 | Leucine Leu | 2,60 | 1,85 |
| 9 | Lysine Lys | 0,39 | 0,3 |
| 10 | Methionine Met | 0,25 | 0,24 |
| 11 | Phenylalanine Phe | 1,13 | 0,93 |
| 12 | Proline Pro | 0,95 | 1,53 |
| 13 | Serine Ser | 0,34 | 1,32 |
| 14 | Threonine Thr | 0,24 | 0,6 |
| 15 | Tryptophan Trp | 0,00 | 0,03 |
| 16 | Valine Val | 0,84 | 0,53 |
| 17 | Cysteine Cys | 0,00 | 0,17 |
| 18 | Tyrosine Tyr | 0,60 | 0,88 |
| | **Total** | **12,49** | **16,78** |

The above comparative table shows that the present invention, compared to the composition described in document EP 2537823, has the following differences, due to the substitution of hydrochloric acid:
- It has zero chlorine residues.
- It has 18 amino acids compared to 16 amino acids in the corresponding document.
- It has a total free L-amino acid concentration of 16,78 % w/w compared to 12,49 % w/w, due to a higher degree of hydrolysis.
- It has sulphur, the fourth most important macronutrient after nitrogen, phosphorus and potassium.
- Thanks to sulphur, it has fungicidal activity.
- It has Betaine and Mannitol, osmolytes, whose action is biostimulant against plant stress factors.

In addition, amino acids of plant origin in 16,78 % w/w of the finished product have the following composition:

| Number | Name | International abbreviation | Molecular formula | g/L of product | % (w/v) of product | % w/w of product |
|---|---|---|---|---|---|---|
| 1 | Alanine | Ala | C3H7NO2 | 13,2 | 1,32 | 1,11 |
| 2 | Arginine | Arg | C6H14N4O2 | 6,1 | 0,61 | 0,51 |
| 3 | Aspartic acid | Asp | C4H7NO4 | 21,1 | 2,11 | 1,77 |
| 4 | Cystine | Cys | C6H12N2O4S2 | 2 | 0,2 | 0,17 |
| 5 | Glutamic acid | Glu | C5H10N2O3 | 44,6 | 4,46 | 3,75 |
| 6 | Glycine | Gly | C2H5NO2 | 6,2 | 0,62 | 0,52 |
| 7 | Histidine | His | C6H9N3O2 | 3,8 | 0,38 | 0,32 |
| 8 | Isoleucine | Ile | C6H13NO2 | 5 | 0,5 | 0,42 |
| 9 | Leucine | Leu | C6H13NO2 | 22 | 2,2 | 1,85 |
| 10 | Lysine | Lys | C6H14N2O2 | 3,6 | 0,36 | 0,3 |
| 11 | Methionine | Met | C5H11NO2S | 2,9 | 0,29 | 0,24 |
| 12 | Phenylalanine | Phe | C9H11NO2 | 11,1 | 1,11 | 0,93 |
| 13 | Proline | Pro | C5H9NO2 | 18,2 | 1,82 | 1,53 |
| 14 | Serine | Ser | C3H7NO3 | 15,7 | 1,57 | 1,32 |
| 15 | Threonine | Thr | C4H9NO3 | 7,1 | 0,71 | 0,6 |
| 16 | Tryptophan | Trp | C11H12N2O2 | 0,3 | 0,03 | 0,03 |
| 17 | Tyrosine | Tyr | C9H11NO3 | 10,5 | 1,05 | 0,88 |
| 18 | Valine | Val | C5H11NO2 | 6,3 | 0,63 | 0,53 |
| | **TOTAL** | | | 199,6 | 19,96 | **16,78** |

The product produced by the present method can be used as a growth supplement (nutrient-fertilizer) for all plants such as cereals, cotton, tobacco, corn, which belong to the large crop plants, trees such as apples (yarrow), walnuts (acorns), oranges (citrus), cherries (stone fruit), various fruit trees and shrubs, such as grapes and olives and in floriculture. It contributes to the rapid absorption of nutrients in fruit and vegetables, whether grown in greenhouses or not and in all types of indoor and outdoor plants/flowers.

Thanks to its hydrolyzed proteins of vegetable origin and its elemental sulphur content, it contributes to environmental and climate protection, long-term soil fertility and a high level of biodiversity.

## Claims

1. A method of producing a plant biostimulant with free amino acids, enriched with elemental sulfur, **characterized by** the following steps, wherein corn gluten 18% w/w, wheat gluten 5.5% w/w, soy protein 6.5% w/w, sulfuric acid 18% w/w. and water 52 % w/w, are placed in a reactor without pre-treatment, the materials remain in the reactor under conditions of continuous, constant agitation, at temperatures ranging from 107° to 113° Celsius, under a pressure ranging from 3,8 to 4,2 bar and for a period of 14 to 18 hours.

2. A method of producing plant biostimulant with free amino acids enriched with elemental sulfur, according to claim 1, **characterized in that** the materials remain in the reactor under conditions of continuous, constant agitation, preferably at a temperature of 110° Celsius, at a pressure of 4 bar for a period of 16 hours.

3. Plant bioreactor with free amino acids, enriched with elemental sulphur, produced according to one of claims 1 and 2, **characterized in that** it is produced with the following materials:
- corn gluten in a proportion of from 15% to 21% w/w,
- wheat gluten in a proportion of 3,5 % to 7,5 % w/w,
- soy protein in a percentage of 4,5% to 8,5% w/w,
- sulphuric acid in a proportion of 16% to 20% w/w,
- water in a proportion of 50% to 54% by weight,
wherein the final composition of the product is:
- Amino acids 16.78% w/w of the product
- Peptides 1.95% w/w of the product
- Sugars, hydrolysis residues 28.55% w/w of the product
- Water 52.7% w/w of the product,
with a density of 1,2 g/ml,
and from the fact that the 18 amino acids of vegetable origin in 16,78 % w/w of the final product have the following composition
Alanine (Ala) 1,11 % w/w of the final product
Arginine (Arg) 0,51 % w/w of the final product
Aspartic acid (Asp) 1,77 % by weight of the final product
Cystine (Cys) 0,17 % w/w of the final product
Glutamic acid (Glu) 3,75 % w/w of the final product
Glycine (Gly) 0,52% w/w of the final product
Histidine (His) 0,32% w/w of the final product
Isoleucine (Ile) 0,42 % w/w of the final product
Leucine (Leu) 1,85 % by weight of the final product
Lysine (Lys) 0,3 % w/w of the final product
Methionine (Met) 0,24 % w/w of the final product
Phenylalanine (Phe) 0,93 % w/w of the final product
Proline (Pro) 1,53 % w/w of the final product
Serine (Ser) 1,32 % w/w of the final product
Threonine (Thr) 0,6 % w/w of the final product
Tryptophan (Trp) 0,03% w/w of the final product
Tyrosine (Tyr) 0,88 % w/w of the final product
Valine (Val) 0,53% w/w of the final product.

4. A plant biostimulant with free amino acids, enriched with elemental sulfur, according to claim 3, wherein it is preferably produced with 18% w/w corn gluten, 5.5% w/w wheat gluten, 6.5% w/w soy protein, 18% w/w sulfuric acid and water 52% w/w.
